# EUROPÄISCHE PATENTSCHRIFT

(11) **EP 2 303 701 B1**
(45) Veröffentlichungstag und Bekanntmachung des Hinweises auf die Patenterteilung: **27.02.2013**
(21) Anmeldenummer: 09765598.9
(22) Anmeldetag: 16.06.2009
(51) Int. Cl.: B65B 3/00, B65B 55/00, B65B 7/28, A61M 5/28

(54) **VERFAHREN ZUM BEFÜLLEN VON DOPPELKAMMERSYSTEMEN IN VORSTERILISIERBAREN TRÄGERSYSTEMEN**
METHOD FOR FILLING DUAL-CHAMBER SYSTEMS IN PRE-STERILIZABLE CARRIER SYSTEMS
PROCÉDÉ POUR REMPLIR DES SYSTÈMES À CHAMBRE DOUBLE DANS DES SYSTÈMES SUPPORTS PRÉSTÉRILISABLES

(30) Priorität: 19.06.2008 DE 102008030268
(43) Veröffentlichungstag der Anmeldung: 06.04.2011
(60) Teilanmeldung: 12005579.3
(73) Patentinhaber: Arzneimittel GmbH Apotheker Vetter & Co. Ravensburg, 88212 Ravensburg (DE)
(72) Erfinder: BÖTTGER, Frank, 88214 Ravensburg (DE); BÖBST, Benjamin, 88441 Mittelbiberach (DE)
(74) Vertreter: Gleiss, Alf-Olav
(86) Internationale Anmeldenummer: PCT/EP2009/004313
(87) Internationale Veröffentlichungsnummer: WO 2009/153018

(56) Entgegenhaltungen:
- EP-A- 0 440 846
- DE-A1- 10 341 978
- US-B1- 6 189 292

## Beschreibung

Die Erfindung betrifft ein Verfahren zum Befüllen von Doppelkammersystemen in vorsterilisierbaren Trägersystemen gemäß den Ansprüchen 1 und 2.

Vorsterilisierbare Trägersysteme und Verfahren zu ihrer Befüllung sind bekannt. Ein bekanntes Trägersystem umfasst üblicherweise gewaschene, silikonisierte und sterilisierte Spritzen, die nach dem Wasch- und Silikonisierungsschritt in einem Magazin platziert werden. Das Magazin - auch Nest genannt - wird anschließend in einen Behälter eingebracht, der dann mit einem Verschlusselement, vorzugsweise einer gasdurchlässigen Membranfolie, versiegelt und über geeignete SterilisationsVertahren sterilisiert wird. Hierbei kommt häufig eine Ethylenoxid-Begasung zur Anwendung. Dadurch, dass das Verschlusselement gasdurchlässig ist, kann das Sterilisationsgas in das Innere des Behälters eindringen und auch den Inhalt des Behälters, also die gewaschenen und silikonisierten Spritzen sowie das diese umfassende Magazin sterilisieren. Der Behälter muss nach dem Sterilisierungsschritt nicht wieder geöffnet werden und kann unmittelbar in der vorliegenden Form an einen Kunden ausgeliefert beziehungsweise einer Abfüllinie zugeführt werden. Das gasdurchlässige Verschlusselement besitzt nämlich eine Filterwirkung dergestalt, dass es zwar durchlässig für ein Sterilisationsgas ist, den Behälter aber dicht und steril gegenüber Keimen, Viren und Bakterien, abschließt. Solange der Behälter verschlossen bleibt, ist demnach die Sterilität seines Inhalts gewährleistet. Beim Kunden, der typischerweise eine Abfüllanlage zum Befüllen der Spritzen oder sonstigen von dem Behälter umfassten Hohlkörper mit pharmazeutischem Inhalt betreibt, wird der Behälter geöffnet, die Hohlkörper werden befüllt und verschlossen, woraufhin auch der Behälter wieder verschlossen und zum Endkunden transportiert werden kann. Selbstverständlich können die gefüllten und verschlossenen Hohlkörper auch aus dem Behälter entnommen und in anderen Packungseinheiten an den Endkunden weitergereicht werden. Wesentlich bei den genannten vorsterilisierten Trägersystemen und den Verfahren zu ihrer Befüllung ist, dass: eine standardisierte Verpackungsform eingesetzt wird, die in Zusammenhang mit genormten Abfülllinien verwendbar ist. Die zu befüllenden Hohlkörper brauchen daher vor dem Abfüllen nicht aus dem Behälter entnommen zu werden, wodurch ein aufwändiger Arbeitsschritt entfällt. Weiterhin ist vorteilhaft, dass die Hohlkörper gemeinsam in bereits abgepackter Form sterilisiert werden können, wonach ein Versand oder eine Weiterverarbeitung erfolgen kann, ohne dass aufwändige Zwischenschritte wie eine Neuverpackung in eine vorsterilisierte weitere Verpackungseinheit oder ein Umpacken nötig ist. Auf Seiten eines produzierenden pharmazeutischen Unternehmens, das die Abfüllung vomimmt, kann ein Reinraum beziehungsweise Arbeitsschritt zur Vorbereitung der Hohlkörper entfallen, da diese befüllfertig angeliefert werden.

Die Fertigung und/oder Vorbereitung der Hohlkörper kann auch als in-line-Prozess mit der Abfüllung erfolgen, wenn ein Heißlufttunnel zwischen der Sterilisationseinrichtung und dem Reinraum, in dem die Abfüllung stattfindet; vorgesehen ist.

Die bekannten vorsterilisierbaren Trägersysteme und die Verfahren zu ihrer Befüllung sind allerdings lediglich für Einkammersysteme, also Einkammerspritzen, Einkammerkarpulen oder Phiolen ausgelegt. Um Doppelkammersysteme wie Doppelkammerspritzen oder -karpulen zu befüllen, sind also nach wie vor aufwändigere Verfahren und Trägervorrichtungen notwendig.

Werden Doppelkammersysteme in herkömmlichen Befüllungsverfahren in Zusammenhang mit einem Gefriertrocknungsschritt für eine in einer der beiden Kammern befindliche Lösung verwendet, kommen spezielle Verschlusselemente - so genannte Lyo-Verschlüsse - zur Anwendung. Jedem einzelnen Doppelkammersystem wird dabei ein solches Verschlusselement zugeordnet. Diese Lyo-Verschlüsse weisen zwei Rastpositionen auf dem Doppelkammersystem auf: In einer ersten Rastposition dichten sie das Doppelkammersystem auf eine Weise ab, dass ein Gasaustausch zwischen dem Inneren der mit dem Verschlusselement verschlossenen Kammer und der Umgebung erfolgen kann. In einer zweiten Rastposition verschließt das Verschlusselement die Kammer vollständig. In herkömmlichen Verfahren sind die Doppelkammersysteme in schwere, wiederverwendbare Metallmagazine einsortiert. Diese haben den Nachteil, aufgrund ihres großen Gewichtes umständlich handhabbar zu sein. Weiterhin müssen sie vor jeder Verwendung umständlich gereinigt und sterilisiert, typischerweise autoklaviert werden.

Aufgabe der Erfindung ist es daher, ein Verfahren zum Befüllen von mindestens einem Doppelkammersystem In Zusammenhang mit einem Gefriertrocknungsschritt unter Verwendung von mindestens einem als Lyo-Verschluss ausgebildeten Verschlusselement in mindestens einem vorsterilisierbaren Trägersystem zu schaffen.

Die der Erfindung zugrunde liegende Aufgabe wird gelöst durch ein Verfahren mit den Merkmalen des Anspruchs 1.

Dieses ist durch die folgenden Schritte gekennzeichnet: Es wird mindestens ein sterilisiertes Doppelkammersystem mit jeweils einem die beiden Kammern voneinander trennenden Trennelement in einem Magazin, weiches das mindestens eine Doppelkammersystem, vorzugsweise eine Anzahl derartiger Systeme aufnimmt, bereitgestellt, wobei das Magazin in einem mit einem Verschlusselement versiegelten Behälter angeordnet ist. Der versiegelte Behälter wird in einen Reinraum eingeschleust. Dort wird er geöffnet, und es wird eine erste Kammer des: mindestens einen Doppelkammersystems befüllt. Diese erste Kammer wird mit einem gasdurchlässigen Verschlusselement verschlossen, und das in der ersten Kammer enthaltene Material wird gefriergetrocknet. Die erste Kammer wird mit einem Verschlusselement verschlossen. Eine zweite Kammer des mindestens einen Doppelkammersystems wird befüllt. Es wird auch die zweite Kammer verschlossen, und das mindestens eine gefüllte Doppelkammersystem wird aus dem Reinraum ausgeschleust. Durch die Verwendung von standardisierten vorsterilisierbaren Trägersystemen wird ein produzierendes pharmazeutisches Unternehmen von der aufwändigen Vorbereitung der Hohlkörper entlastet, und der Einsatz genormter Abfülllinien wird möglich. Vorzugsweise ist das bereitgestellte Doppelkammersystem gewaschen und silikonisiert.

Die der Erfindung zugrurideliegende Aufgabe wird auch gelöst durch ein Verfahren mit den Merkmalen des Anspruchs 2.

Dieses umfasst die folgenden Schritte: Es wird mindestens ein sterilisiertes Doppelkammersystem bereitgestellt, das ein die beiden Kammern voneinander trennendes Trennelement aufweist. Ein Magazin nimmt das mindestens eine Doppelkammersystem auf, wobei das Magazin in einem Behälter angeordnet ist, der mit einem Verschlusselement versiegelt ist. Der Behälter wird in einen Reinraum eingeschleust. Er wird geöffnet, und die erste Kammer des mindestens einen Doppelkammersystems wird befüllt. Die erste Kammer wird mit einem gasdurchlässigen Verschlusselement verschlossen. Der Behälter wird mit einem gasdurchlässigen Verschlusselement verschlossen. Es folgt ein Verfahrensschritt, in dem das in der ersten Kammer des mindestens einen Doppelkammersystems enthaltene Material gefriergetrocknet wird. Hierbei sublimiert der Lösungsmitteldampf durch die gasdurchlässigen Verschlusselemente der ersten Kammer des mindestens einen Doppelkammersystems und das gasdurchlässige Verschlusselement des Behälters hindurch. Nach der Gefriertrocknung wird die erste Kammer mit einem Verschlusselement verschlossen. Der Behälter wird geöffnet, und es wird eine zweite Kammer des mindestens einen Doppelkammersystems befüllt und verschlossen. Das mindestens eine Doppelkammersystem wird aus dem Reinraum ausgeschleust. Vorzugsweise ist das bereitgestellte Doppelkammersystem gewaschen und silikonisiert.

Es wird auch ein Verfahren bevorzugt, das sich dadurch auszeichnet, dass das Magazin, welches das mindestens eine Doppelkammersystem aufnimmt, Kunststoff umfasst, vorzugsweise aus Kunststoff besteht. Hierdurch ist das Magazin sehr leicht und damit auch leicht zu handhaben. Es kann weiterhin als Produkt zur einmaligen Verwendung bestimmt sein, so dass es im Anschluss an seine Verwendung entsorgbar ist. Es entfallen somit die in bekannten Träger-systemen üblichen schweren Metallmagazine, die zum Einen schwer zu handhaben sind und zum Anderen autoklaviert werden müssen, um sie steril zu halten. blei den hier beschriebenen Trägersystemen wird dagegen mit jeder neuen Lieferung ein neues Kunststoffmagazin mitgeliefert, das genau einem Doppelkammersystem oder insbesondere einer Charge von Doppelkammersystemen zugeordnet ist und nach seiner Verwendung entsorgt wird. Neben dem Wegfall aufwändiger Arbeitsschritte führt dies insbesondere dazu, dass eine hinsichtlich ihrer Sterilität gut reproduzierbare Handhabung von Doppelkammersystemen möglich ist.

Es wird auch ein Verfahren bevorzugt, bei dem der Behälter Kunststoff umfasst, vorzugsweise aus Kunststoff besteht. Auch hier ist vorzugsweise vorgesehen, dass der Behälter einmalig verwendet und nach seiner Verwendung entsorgt wird. Jeder Charge von Doppelkammersystemen ist ein Behälter eindeutig zugeordnet, so dass auch hier die Sterilität der Chargen mit sehr guter Reproduzierbarkeit gewährleistet ist.

Bevorzugt wird auch ein Verfahren, bei dem insbesondere der Kunststoff, den der Behälter umfasst, beziehungsweise aus dem der Behälter vorzugsweise besteht, elastisch verformbar ist. In herkömmlichen Verfahren werden die Lyo-Verschlüsse nach dem Gefriertrocknen dadurch verschlossen, dass der vertikale Abstand der Regalböden der Vorrichtung zum Gefriertrocknen auf eine Weise verringert wird, dass die Lyo-Verschlüsse von ihrer ersten Rastposition in ihre zweite Rastposition gedrängt werden. Dies ist möglich, da die bekannten Trägersysteme aus Metall die Doppelkammersysteme nur seitlich umfassen und keine Höhe aufweisen, die größer ist als die vertikale Ausdehnung oder Doppelkammersysteme. Im Gegensatz dazu ist der Behälter eines vorsterilisierbaren Trägersystems so ausgebildet, dass seine Wände eine größere Höhe aufweisen als die Doppelkammersysteme, sodass diese vollständig und sicher in dem Behälter eingebettet sind. Dies bedeutet, dass bei einem starren Behälter die Lyo-Verschlüsse durch eine Vorrichtung, die in den Behälter eingreifen kann, in ihre zweite Rastposition gedrängt werden müssten. ist der Behälter dagegen aus einem elastisch verformbaren Kunststoff gebildet, kann das bekannte Verschließverfahren für die Lyo-Verschlüsse angewendet werden. Bewegen sich nämlich die Regalböden des Gefriertrockners so aufeinander zu, dass ihr vertikaler Abstand verringert wird, stauchen sie den elastisch verformbaren Behälter entlang seiner vertikalen Erstreckung, sodass die Lyo-Verschlüsse in ihre zweite Rastposition drangbar sind. Ein vorsterilisierbares Trägersystem, das einen Behälter aus einem elastisch verformbaren Kunststoff aufweist, ermöglicht es also, die Lyo-Verschlüsse der Doppelkammersysteme auf sehr einfache und bekannte Weise in eine Position zu verlagem, in der sie die erste Kammer der Doppelkammersysteme dicht abschließen.

Bevorzugt wird auch ein Verfahren, bei dem der Behälter nach dem Befüllen der ersten Kammer des mindestens einen Doppelkammersystems und dem Verschließen der ersten Kammer und des Behälters mit einem gasdurchlässigen Verschlusselement, zunächst aus dem Reinraum ausgeschleust und in eine außerhalb des Reinraums angeordnete Vorrichtung zum Gefriertrocknen eingebracht wird. Dort findet die Gefriertrocknung statt, nach deren Ende der Behälter aus der Vorrichtung entnommen und wiederum in einen Reinraum eingeschleust wird. Wird das Verfahren um diesen Schritt erweitert, ist es möglich, die aseptische Abfüllung des pharmazeutischen Inhalts vollständig von der Gefriertrocknung zu trennen, wobei diese nicht unter aseptischen Bedingungen stattfinden muss. Dies wird möglich, weil der Behälter mit einem gasdurchlässigen Verschlusselement versehen ist, das zwar den sublimierten Lösungsmitteldampf während des Gefriertrockenprozesses vom Inneren des Behälters nach Außen durchlässt, aber Keime, Viren und Bakterien davon abhält, in den Behälter einzudringen. Das Innere des Behälters bleibt also aseptisch, auch wenn die Umgebung in dem Gefriertrockner nicht steril gehalten wird. Auf diese Weise können aufwändige Reinigungs- und Desinfizierungsschritte für den Gefriertrockner entfallen, und dieser braucht auch nicht innerhalb des Reinraums angeordnet zu sein.

In diesem Zusammenhang wird auch ein Verfahren bevorzugt, das sich dadurch auszeichnet, dass die Vorrichtung zum Gefriertrocknen selbst nicht steril und/oder aseptisch ist. Wie gesagt wird dies möglich durch das Verschließen des Behälters mit einem gaspermeablen aber für Viren, Bakterien und Keime nicht durchlässigen Verschlusselement.

Weitere vorteilhafte Ausgestaltungen bezüglich der beanspruchten Verfahren ergeben sich aus den Unteransprüchen.

Es wird außerdem ein nicht zur Erfindung gehörendes, vorsteritisierbares Trägersystem für mindestens ein Doppelkammersystem beschrieben.

Dieses umfasst mindestens ein sterilisiertes, vorzugsweise gewaschenes und silikonisiertes Doppelkammersystem, welches ein Trennelement aufweist, das die beiden Kammern voneinander abtrennt. Weiterhin umfasst das vorsterillsierbare Trägersystem ein Magazin, das zur Aufnahme des mindestens einen Doppelkammer-systems dient. Es umfasst auch einen Behälter. Das Magazin, welches das mindestens eine Doppelkammersystem aufnimmt, ist in dem Behälter anordenbar, wobei dieser mit einem Verschlusselement versiegelt werden kann. Es entsteht so ein geschlossener Behälter, in dem ein Magazin angeordnet ist, welches mindestens ein vorzugsweise gewaschenes und silikonisiertes, sterilisiertes Doppelkammersystem umfasst. Besonders bevorzugt wird, wenn der gesamte Behälter in seinem Inneren sterilisiert ist. Durch die Versiegelung können solche mit mindestens einem Doppelkammersystem bestückte vorsterilisierbare Trägersysteme auf Vorrat produziert und gelagert werden, wobei der Inhalt steril bleibt.

Es wird auch ein nicht zur Erfindung gehörendes vorsterilisierbares Trägersystem beschrieben, bei dem das Magazin Kunststoff umfasst, vorzugsweise aus Kunststoff besteht. In diesem Fall ist das Magazin besonders leicht und außerdem nach der Verwendung des vorsterilisierbaren Trägersystems entsorgbar, so dass aufwändige Reinigungs- oder Autoklavierschritte entfallen. Außerdem ist jede Charge von Doppelkammersystemen genau einem Magazin zugeordnet, so dass eine hinsichtlich der Sterilität sehr gut reproduzierbare Handhabung möglich ist.

Es wird auch ein nicht.zur Erfindung gehörendes vorsterilisierbares Trägersystem beschrieben, bei dem der Behälter Kunststoff umfasst, vorzugsweise aus Kunststoff besteht. Auch in diesem Fall ist der Behälter zur einmaligen Verwendung vorgesehen, so dass jede Charge von Doppelkammersystemen genau einem Behälter zugeordnet ist. Auch dies erhöht die Reproduzierbarkeit der Handhabung im Hinblick auf ihre Sterilität.

Es wird auch ein nicht zur Erfindung gehörendes vorsterilisierbares Trägersystem beschrieben, bei dem insbesondere der Kunststoff, den der Behälter umfasst beziehungsweise aus dem der Behälter vorzugsweise besteht, elastisch verformbar ist. Hierdurch ist es möglich, Lyo-Verschlüsse, die sich auf den Doppelkammersystemen in einer ersten Rastposition befinden, nach dem Gefriertrocknen durch Verringerung des vertikalen Abstands zwischen den Regalböden der Vorrichtung zum Gefriertrocknen in eine zweite Rastposition zu drängen, in der sie die erste Kammer der Doppelkammersysteme dicht verschließen. Der Behälter, dessen Wände eine größere Höhe aufweisen als die Doppelkammersysteme, sodass sie diese vollständig umgeben, wird dabei entlang seiner vertikalen Erstreckung gestaucht. So ist es möglich, die Lyo-Verschlüsse auf sehr einfache und bekannte Weise in eine zweite Rastposition zu drängen, in der sie die erste Kammer der Doppelkammersysteme dicht verschließen.

Weiterhin wird ein nicht zur Erfindung gehörendes vorsterilisierbares Trägersystem beschrieben, bei dem das Verschlusselement für den Behälter gasdurchlässig ist. In diesem Fall kann der bereits mit dem Magazin und den Doppelkammersystemen bestückte Behälter beim Hersteller verschlossen und anschließend sterilisiert werden, indem das zur Sterilisation bestimmte Gas durch das gasdurchlässige Verschlusselement in das Innere des Behälters eindringt. Nach der Sterilisation ist es nicht mehr nötig, den Behälter zu öffnen, und er kann sofort weiter transportiert werden, beispielsweise zu einer Abfülllinie. Dadurch, dass der Behälter schon während der Sterilisation endgültig geschlossen ist, kann nicht durch ein nachfolgendes Öffnen oder Verschließen keimhaltiges Material von Außen in das Innere des Behälters eindringen. Dabei spricht das Wort gasdurchlässig an, dass das Verschlusselement zwar Gase und Dämpfe durchlässt, aber Keime, Viren oder Bakterien daran hindert, in das Innere des Behälters einzudringen.

Die Erfindung wird im Folgenden anhand der Zeichnungen näher erläutert. Es zeigen:
- Figur 1: eine schematische Ansicht eines nicht zur Erfindung gehörenden vorsteritisierbaren Trägersystems;
- Figur 2: eine schematische Darstellung des Schritts der Befüllung einer, ersten Kammer der Doppelkammersysteme in einem erfindungsgemäßen Verfahren;
- Figur 3: eine schematische Ansicht des Verschließens der ersten Kammer der Doppelkammersysteme mit einem gasdurchlässigen Verschlusselement in dem Verfahren;
- Figur 4: eine schematische Darstellung des Befüllens einer zweiten Kammer der Doppelkammersysteme in dem Verfahren; und
- Figur 5: das Verschließen der zweiten Kammer der Doppelkammersysteme in dem Verfahren.

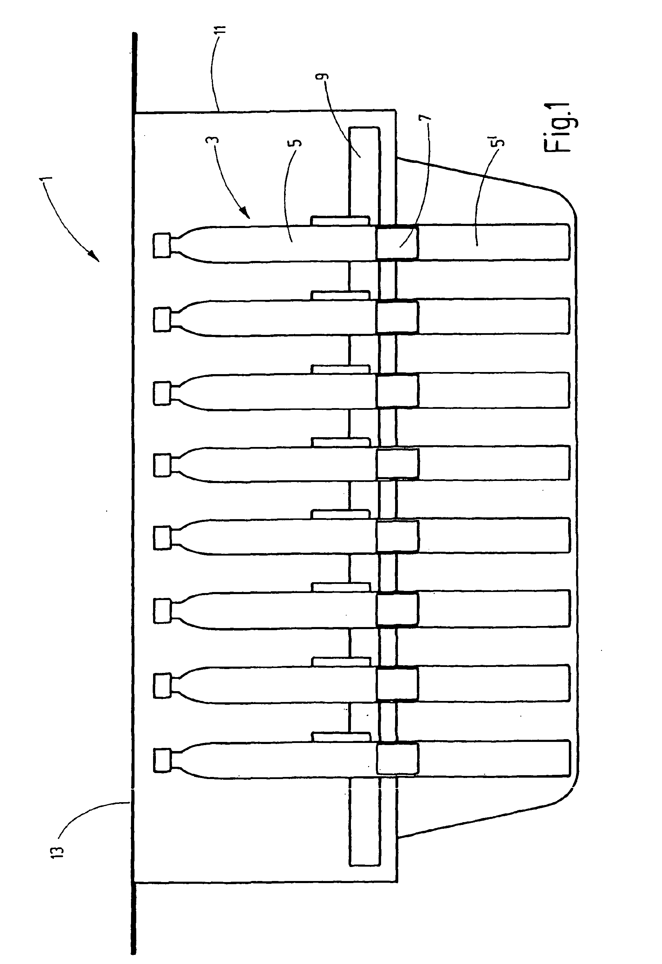

Figur 1 zeigt schematisch ein nicht zur Erfindung gehörendes Beispiel eines vorsterilisierbaren Trägersystems 1. Es umfasst mindestens ein sterilisiertes, vorzugsweise gewaschenes und silikonisiertes Doppelkammersystem !3 mit zwei Kammern 5, 5', die durch ein Trennelement 7 voneinander separiert sind. Die Doppolkammersysteme 3 werden von einem Magazin 9 aufgenommen, das seinerseits in einem Behälter 11 angeordnet sein kann. Dieser ist mit einem Verschlusselement 13 versiegelt.

Der Behälter 11 kann Kunststoff umfassen, vorzugsweise besteht er aus Kunststoff. Auch das Magazin 9 kann Kunststoff umfassen und besteht vorzugsweise aus Kunststoff. Beide Elemente sind auf diese Weise zur einmaligen Verwendung bestimmbar, so dass jede Charge von Doppelkammersystemen 3 jeweils ein Magazin 9 und ein Behälter 11 zugeordnet ist. Hierdurch entfallen die in bekannten Verfahren notwendigen Reinigungs- und Autoklavierschritte, die zur Sterilisation der wieder verwendbaren Metallmagazine vorgesehen sind. Außerdem sind beide aus Kunststoff bestehenden Elemente leicht zu handhaben, insbesondere leichter als die bekannten schweren Trägersysteme aus Metall.

Das Verschlusselement: 13 für den Behälter 11 ist vorzugsweise gasdurchlässig ausgebildet, so dass der vollständig bestückte und versiegelte Behälter 11 in geschlossenem Zustand sterilisiert werden kann, indem er in eine Atmosphäre eingebracht wird, die ein zur Sterilisation bestimmtes Gas oder einen zur Sterilisation bestimmten Dampf umfasst. Das Gas oder der Dampf können durch das Verschlusselement 13 in das Innere des Behälters 11 eindringen und so insbesondere auch den Innenraum des Behälters 11 sowie die darin enthaltenen Doppelkammersysteme 3 und das Magazin 9 sterilisieren.

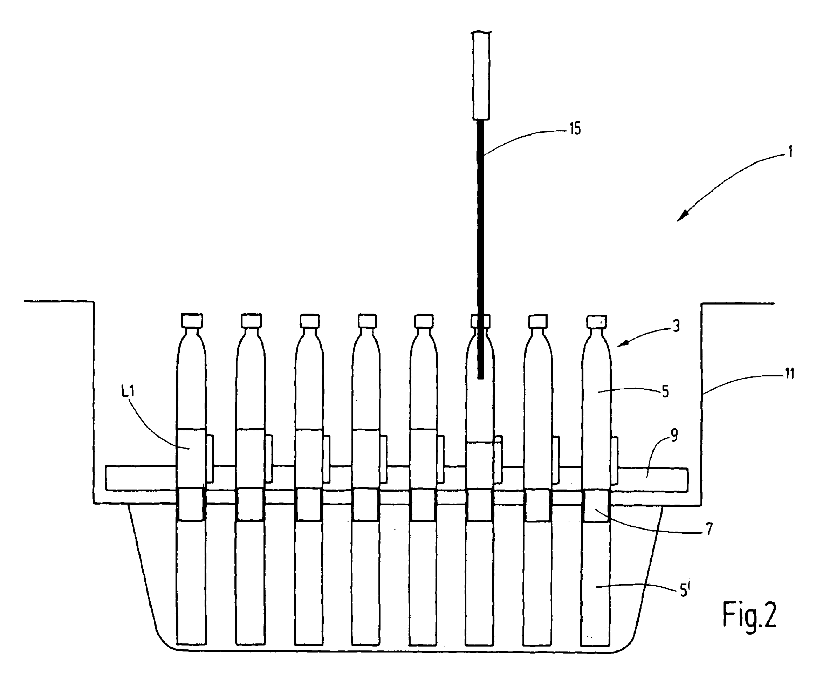

Die verschiedenen Verfahren werden nun anhand der Figuren 2 bis 5 näher erläutert.

Zunächst wird das vorsterilisierbare Trägersystem 1 bereitgestellt, und in einen Reinraum eingeschleust. Sodann wird das Verschlusselement 13 entfernt, so dass die Doppelkammersysteme 3 zugänglich sind.

Figur 2 zeigt den Schritt des Befüllens einer ersten Kammer 5 der Doppelkammersysteme 3. Gleiche und funktionsgleiche Elemente sind mit gleichen Bezugszeichen versehen, so dass insofern auf die vorangegangene Beschreibung verwiesen wird. Es ist eine Abgabevorrichtung 15 vorgesehen, durch die eine erste Lösung L1 eines Wirk- und/oder Hilfsstoffs in eine erste Kammer 5 der Doppelkammersysteme 3 einbringbar ist.

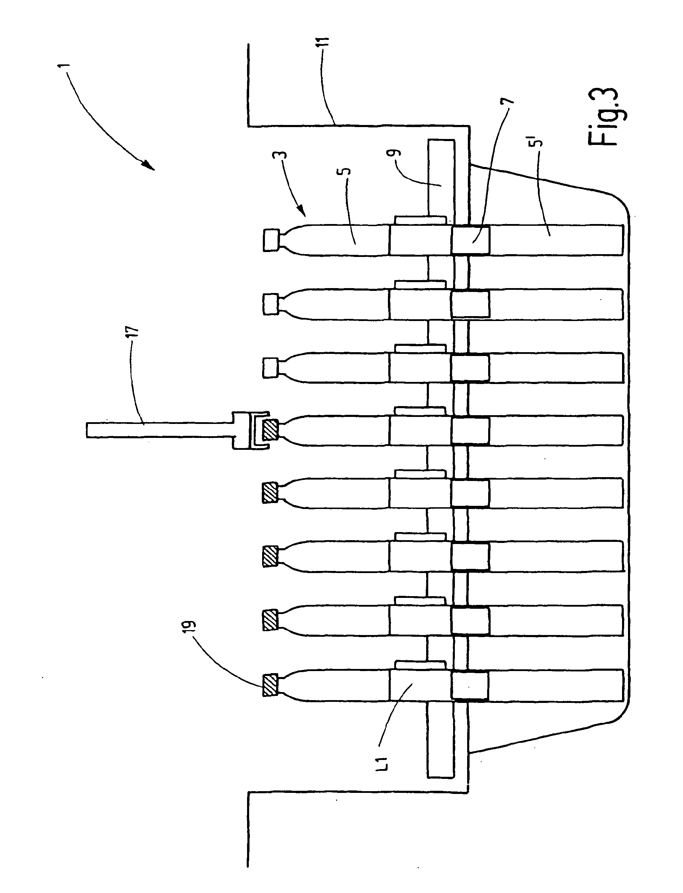

Nach dem Befüllen der ersten Kammer 5 der Doppelkammersysteme 3 kann die erste Kammer verschlossen werden, wie es in Figur 3 gezeigt ist. Gleiche und funktionsgleiche Elemente sind mit gleichen Bezugszeichen versehen, so dass insofern auf die vorangegangene Beschreibung verwiesen:wird. Es ist eine erste Verschließvorrichtung 17 vorgesehen, mit der die erste Kammer 5 der Doppelkammersysteme 3 mit jeweils einem Verschluss 19 gasdurchlässig verschließbar ist. Der Verschluss 19 ist als Lyo-Verschluss ausgebildet und wird von der ersten Schließvorrichtung 17 in eine erste Rastposition gebracht, sodass die erste Kammer 5 der Doppelkammersysteme gasdurchlässig verschlossen ist.

Der Behälter 11 kann nun in eine Vorrichtung zum Gefriertrocknen eingebracht werden. Da er offen ist, muss sich die Vorrichtung zum Gefriertrocknen ebenfalls in einem Reinraum befinden. Es ist möglich, den offenen Behälter 11 durch einen Heißlufttunnel von dem ersten Reinraum in einen zweiten Reinraum zu schleusen, in dem sich die Einrichtung zum Gefriertrocknen befindet. Diese kann sich allerdings auch in dem ersten Reinraum befinden, sodass kein Heißlufttunnel vorgesehen sein muss. Während des Gefriertrocknens sublimiert das in der ersten Kammer 5 enthaltene Lösungsmittel durch den gasdurchlässigen Verschluss 19 der ersten Kammer hindurch. Nachdem der Gefriertrocknungsschritt abgeschossen ist, werden die Verschlüsse 19 aus ihrer ersten Rastposition in eine zweite Rastposition verlagert, in der sie die erste Kammer 5 der Doppelkammersysteme 3 dicht verschließen. Dies geschieht vorzugsweise noch innerhalb der Einrichtung zum Gefriertrocknen, indem deren Regalböden iin vertikaler Richtung so aufeinanderzubewegt werden, dass ihr Abstand sich verringert und die Verschlüsse 19 hierdurch in ihre zweite Rastposition gedrängt werden. Hierzu kann vorgesehen sein, dass der Kunststoff, den der Behälter 11 umfasst und aus dem dieser vorzugsweise besteht, elastisch verformbar ist, sodass er in vertikaler Richtung gestaucht werden kann. Selbstverständlich kann der Behälter 11 auch zunächst der Vorrichtung zum Gefriertrocknen entnommen werden, woraufhin dann in einem zweiten Schritt die Verschlüsse 19 durch eine beliebige Vorrichtung, die wahlweise die Wände des Behälters 11 staucht oder in das Innere des Behälters 11 eingreift, in ihre zweite Rastposition gedrängt werden.

Der Behälter 11 kann auch vor dem Gefriertrocknungsschritt mit einem gasdurchlässigen Verschlusselement 13, vorzugsweise einer gasdurchlässigen Membranfolie verschlossen werden. Der auf diese Weise versiegelte Behälter 11 kann in eine Vorrichtung zum Gefriertrocknen eingebracht werden, wo das in der ersten Kammer 5 enthaltene Lösungsmittel durch den gasdurchlässigen Verschluss 19 der ersten Kammer und das gasdurchlässige Verschlusselement 13 des Behälters hindurch sublimiert so dass letztendlich der in den Doppelkammersystemen 3 vorliegende Wirk- und/oder Hilfsstoff gefriergetrocknet wird. Da der Behälter 11 durch das gasdurchlässige Verschlusselement 13 hygienisch versiegelt ist, ist es möglich, die Vorrichtung zur Gefriertrocknung außerhalb des Reinraums vorzusehen. Der Behälter 11 kann also aus dem Reinraum ausgeschleust und in eine externe Gefriertrockenvorrlchtung eingebracht werden. Diese muss selbst nicht steril und/oder aseptisch gehalten werden, da keine Keime, Viren oder Bakterien durch das Verschlusselement 13 in das Innere des Behälters 11 gelangen können. So bleiben insbesondere die Doppelkammersysteme 3 steril beziehungsweise aseptisch, auch wenn die Gefriertrocknung in einer nicht-sterilen und/oder aseptischen Umgebung vorgenommen wird.

Nach dem Gefriertrocknen werden die Verschlüsse 19 von ihrer ersten Rastposition in eine zweite Rastposition gedrängt, in der sie die erste Kammer 5 der Doppelkammersysteme 3 dichtend verschließen. Dies geschieht vorzugsweise noch innerhalb der Einrichtung zum Gefriertrocknen. Hierzu ist vorgesehen, dass der Behälter 11 einen elastisch verformbaren Kunststoff umfasst, vorzugsweise aus diesem besteht. Durch eine Verringerung des vertikalen Abstands der Regalböden der Einrichtung zum Gefriertrocknen kann der Behälter 11 so in Richtung seiner vertikalen Erstreckung gestaucht werden. Hierdurch sind die Verschlüsse 19 aus einer ersten Rastposition in eine zweite Rastposition drängbar, in der sie die Doppel-kammersysteme 3 dicht umschließen. Während dieses Vorgangs wird das gasdurchlässige Verschlusselement 13 nicht von dem Behälter 11 gelöst, sodass dieser keimfrei verschlossen bleibt. Selbstverständlich ist es auch möglich, den Behälter 11 zunächst aus der Vorrichtung zum Gefriertrocknen zu entnehmen, und in einem zweiten Schritt die Verschlüsse 19 auf geeignete Weise in ihre zweite Rastposition zu drängen Dieses kann entweder noch außerhalb des Reinraums oder nach einem erneuten Einschleusen in den ersten oder in einen weiteren Reinraum geschehen.

Nachdem die Verschlüsse 19 in ihre zweite Rastposition gedrängt wurden, können sie mit einem Originalitätsverschluss verschlossen werden. Die Verschlüsse 19 bleiben dabei auf den Doppelkammersystemen 3, und der Originalitätsverschluss wird auf die Verschlüsse 19 aufgesetzt. Er dient dazu, bei einer Verwendung der Doppelkammersysteme 3 anzuzeigen, ob die Verschlüsse 19 nach dem endgültigen Verschließen in der Produktionslinie noch einmal geöffnet wurden, oder ob der Originalverschluss erhalten ist. Insofern handelt es sich bei den Originalitätsverschlüssen um Garantieverschlüsse, die einem Verwender die unbeschädigte Dichtigkeit der ersten Kammer 5 der Doppelkammersysteme 3 signalisieren und auf die gleiche Weise auch anzeigen, dass der Inhalt der Kammer 5 nicht kontaminiert oder verändert wurde.

Während des Gefriertrocknens sind die Doppelkammersysteme 3 in dem Behälter 11 eingebettet und vor thermischer Störstrahlung oder anderen Störeinflüssen sicher geschützt.

Nach dem Gefriertrocken und dem Verschließen der ersten Kammer 5 muss der Behälter 11 gegebenenfalls emeut geöffnet werden, damit die Doppelkammersysteme 3 zugänglich sind. Es wird eine zweite Kammer 5' befüllt. Dies ist auf besonders einfache Weise möglich, wenn das Magazin 9 gewendet wird. Es ist in diesem Fall vorgesehen, dass das Magazin 9 die Doppelkammersysteme 3 derart umfasst, dass diese unabhängig von der Orientierung des Magazins 9 sicher in diesem gehalten werden. So ist gewährleistet, dass die Doppelkammersysteme 3 auch beim Wenden des Magazins 9 nicht aus diesem herausrutschen. Nach dem Wenden des Magazins 9 aus diesem herausrutschen. Nach dem Wenden des Magazins 9 wird dieses vorzugsweise wieder in den Behälter 11 eingebracht, wobei nun durch die Öffnung des Behälters 11 eine zweite Kammer 5' der Doppelkammersysteme 3 zugänglich ist.

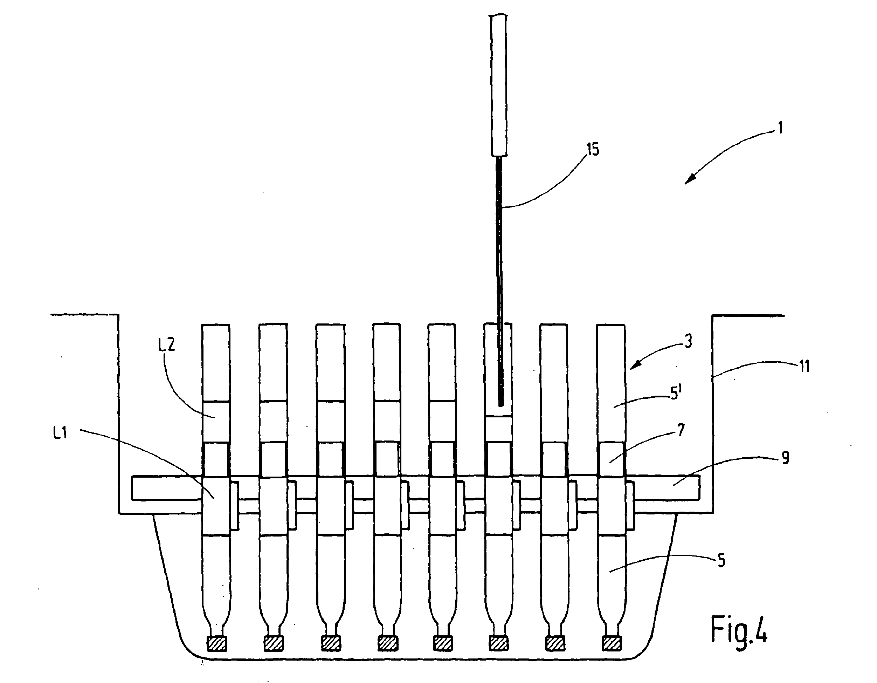

Figur 4 zeigt schematisch das Befüllen der zweiten Kammer 5' der Doppelkammersysteme : 3. Gleiche und funktionsgleiche Elemente sind mit gleichen Bezugszeichen versehen, so dass insofern auf die vorangegangene Beschreibung verwiesen werden kann. Auch hier ist wieder eine Abgabeeinrichtung 15 vorgesehen, durch die ein zweites Medium L2 in die zweite Kammer 5 der Doppelkammersysteme 3 einbringbar ist. Bei dem zweiten Medium L2 kann es sich um die Lösung eines weiteren Wirk- und/oder Hilfsstoffes handeln, es kann sich aber auch um ein - vorzugsweise reines - Lösungsmittel oder um ein Lösungsmittelgemisch handeln.

Nach dem Befüllen der zweiten Kammer 5' der Doppelkammersysteme 3 kann auch diese verschlossen werden.

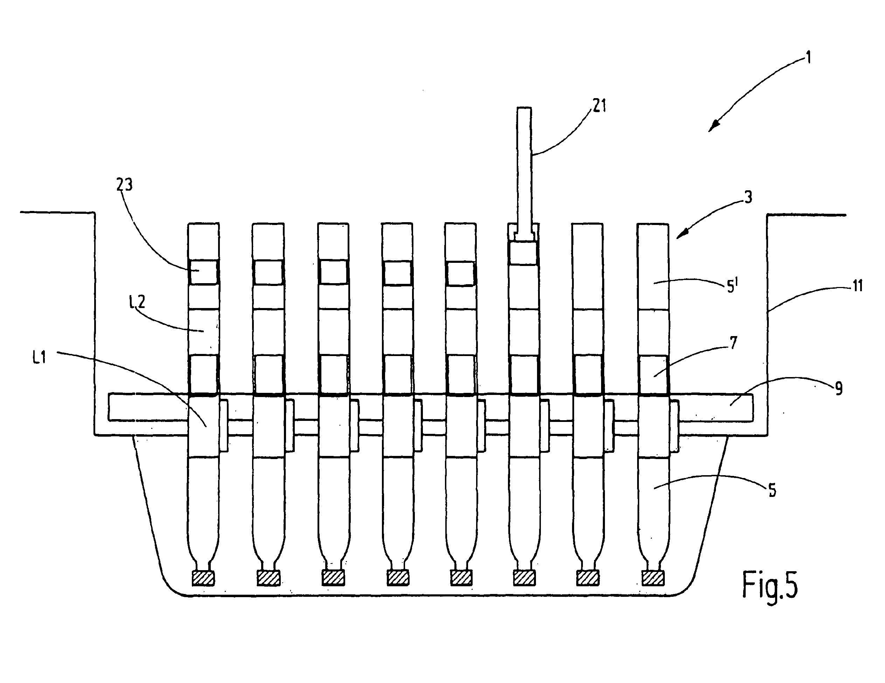

Figur 5 zeigt schematisch den Schritt des Verschließens der zweiten Kammer 5' der Doppelkammersysteme 3. Gleiche und funktionsgleiche Elemente sind mit gleichen Bezugszeichen versehen, sodass insofern auf die vorangegangene Beschreibung verwiesen wird. Die zweite Kammer 5' wird mit Hilfe einer zweiten Verschließvorrichtung 21 mit einem Verschlusselement verschlossen, das hier beispielhaft als Stopfen 23 ausgebildet. Dieser ist vorzugsweise in dem Doppelkammersystem 3 verlagerbar, sodass über ihn Druckkräfte in die zweite Kammer 5' und letztlich in das Trennelement 7 einleitbar sind, die zu einer Aktivierung des Doppelkammersystems 3 führen. Bevorzugt wird ein Stopfen 23, der als Gewindestopfen ausgebildet ist. Er kann so als Kolbenelement wirken, wobei eine nicht dargestellte Kolbenstange mit Hilfe eines Außengewindes mit dem Innengewinde des Gewindestopfens 23 in Eingriff gebracht werden kann. So können auf sehr einfache Weise Druckkräfte in die zweite Kammer 5' und damit mittelbar in das Trennelement 7 eingeleitet werden, die zu einer Aktivierung der Doppetkammersysteme 3 führen.

Nach dem Verschließen der zweiten Kammer 5' kann der Behälter 11 wieder verschlossen. und aus dem Reinraum ausgeschleust werden. Es ist auch möglich, das Verschließen des Behälters 11 zu unterlassen, und wahlweise den Behälter 11 offen aus dem Reinraum auszuschleusen, oder lediglich das Magazin 9 oder sogar die einzelnen Doppelkammersysteme 3 aus dem Reinraum auszuschleusen. Da nämlich beide Kammern 5, 5' der Doppelkammersysteme 3 dicht verschlossen sind, ist es nicht erforderlich, die Doppelkammersysteme 3 weiterhin in einer sterilen und/oder aseptischen Umgebung zu belassen.

Nach allem zeigt sich, dass die erfindungsgemäßen Herstellungsverfahren vorteilhaft gegenüber bekannten Verfahren zur Befüllung von Doppeikammersystemen sind. Erfindungsgemäß ist es für ein produzierendes pharmazeutisches Unternehmen möglich, eine standardisierte Verpackungsform direkt auf genormten Abfülllinien einzusetzen. Hierbei können Produkte, die zur Gefriertrocknung bestimmt sind, auf Anlagen abgefüllt werden, die für vorsterilisierbare Systeme ausgelegt sind. In bekannten Verfahren werden zur Befüllung von Doppelkammersystemen im Zusammenhang mit Materialien, die einer Gefriertrocknung zugeführt werden sollen, schwere und teure metallische Magazine eingesetzt, die wiederverwendet werden und daher aufwändig autoklaviert werden müssen. Im vorliegenden Fall wird anstelle solcher Magazine eine standardisierte Verpackungswird anstelle Solcher Magazine eine standardisierte Verpackungsform während des gesamten Abfüllverfahrens eingesetzt, die vorzugsweise einer einzigen Verwendung zugeführt und danach entsorgt wird. Da das Trägersystem gasdurchlässig, aber für Keime, Viren oder Bakterien undurchdringbar versiegelt werden kann, ist es möglich, die Befüllung und die Gefriertrocknung dezentral zueinander anzuordnen, was es weiterhin ermöglicht, die Gefriertrocknung in einer unsterilen Umgebung durchführen zu können. Der Inhalt des Trägersystems bleibt dabei zu jeder Zeit steril. Das Trägersystem kann auf besonders einfache Weise mit an sich bekannten Verschlüssen 19, die als Lyo-Verschlosse ausgebildet sind, zusammenwirken, wenn zumindest der Behälter 11 einen elastisch verformbaren Kunststoff umfasst beziehungsweise vorzugsweise aus diesem besteht. Hierdurch ist esi möglich, die Vorteile des Trägersystems mit den Vorteilen der an sich bekannten Verschlüsse 19 zu kombinleren. Insbesondere sind diese durch die elastische Verformbarkeit des Behälters 11 sehr einfach und in an sich bekannter Weise aus einer ersten Rastposition in eine zweite Rastposition drängbar, in der sie eine Kammer 5 der Doppelkammersysteme 3 dicht verschließen. Wird der Behälter 11 vor dem Gefriertrocknen mit einem gasdurchlässigen Verschlusselement 13 versiegelt, werden bei dem semiautomatischen, automatischen oder manuellen Be- und Entladen des Gefriertrockners lediglich hygienisch verschlossene Behälter gehandhabt, so dass auch hier ein deutlich geringeres Kontaminationrisiko besteht als dies bei bekannten Verfahren der Fall ist.

## Patentansprüche

1. Verfahren zum Befüllen von Doppelkammersystemen (3) in vorsterilisierbaren Trägersystemen (1), **gekennzeichnet durch** die folgenden Schritte:
- Bereitstellen von mindestens einem sterilisierten Doppelkammersystem (3) mit je einem die beiden Kammern (5,5') voneinander trennenden Trennelement (7) in einem Magazin (9), welches das mindestens eine Doppelkammersystem (3) aufnimmt, wobei das Magazin (9) in einem mit einem Verschlusselement (13) versiegelten Behälter (11) angeordnet ist;
- Einschleusen des Behälters (11) in einen Reinraum;
- Offnen des Behälters (11) und Befüllen einer ersten Kammer (5) des mindestens einen Doppelkammersystems (3);
- Verschließen der ersten Kammer (5) des mindestens einen Doppelkarmmersystems (3) mit einem gasdurchlässigen Verschlusselement (19);
- Gefriertrocknen der in der ersten Kammer (5) des mindestens einen Doppelkammersystems (3) enthaltenen Lösung (L1);
- Verschließen :der ersten Kammer (5) des mindestens einen Doppelkammersystems (3) mit einem Verschlusselement (19);
- Befüllen einer zweiten Kammer (5') des mindestens einen Doppelkammersystems (3);
- Verschließen der zweiten Kammer (5') des mindestens einen Doppelkammersystems (3);
- Ausschleusen aus dem Reinraum.

2. Verfahren zum Befüllen von Doppelkammersystemen (3) in vorsterilisierbaren Trägersystemen (1), **gekennzeichnet durch** die folgenden Schritte:
- Bereitstellen von mindestens einem sterilisierten Doppelkammersystem (3) mit je einem die beiden Kammern (5,5') voneinander trennenden Trennelement (7) in einem Magazin (9), welches das mindestens eine Doppelkammersystem (3) aufnimmt, wobei das Magazin (9) in einem mit einem Verschlusselement (13) versiegelten Behälter (11) angeordnet ist;
- Einschleusen des Behälters (11) in einen Reinraum;
- Offnen des Behälters (11) und Befüllen einer ersten Kammer (5) des mindestens einen Doppelkammersystems (3);
- Verschließen der ersten Kammer (5) des mindestens einen Doppelkammersystems (3) mit einem gasdurchlässigen Verschlusselement (19);
- Verschließen des Behälters (11) mit einem gasdurchlässigen Verschlusselement (13);
- Gefriertrocknen der in der ersten Kammer (5) des mindestens einen Doppelkammersystems enthaltenen Lösung (L1);
- Verschließen der ersten Kammer (5) des mindestens einen Doppelkammersystems (3) mit einem Verschlusselement (19);
- Öffnen des Behälters (11) und Befüllen einer zweiten Kammer (5') des mindestens einen Doppelkammersystems (3);
- Verschließen der zweiten Kammer (5') des mindestens einen Doppelkammersystems (3);
- Ausschleusen aus dem Reinraum.

3. Verfahren nach einem der Ansprüche 1 oder 2, **dadurch gekennzeichnet, dass** das Magazin (9) Kunststoff umfasst, vorzugsweise aus Kunststoff besteht.

4. Verfahren nach einem der vorhergehenden Ansprüche, **dadurch gekennzeichnet, dass** der Behälter (11) Kunststoff umfasst, vorzugsweise aus Kunststoff besteht.

5. Verfahren nach Anspruch 4, **dadurch gekennzeichnet, dass** der Kunststoff elastisch verformbar ist.

6. Verfahren nach einem der Ansprüche 2 bis 5, **dadurch gekennzeichnet, dass** der Behälter (11) nach dem Befüllen der ersten Kammer (5) des mindestens einen Doppelkammersystems (3) und dem Verschließen der ersten Kammer und des Behälters mit einem gasdurchlässigen Verschlusselement (13) aus dem Reinraum ausgeschleust und in eine außerhalb des Reinraums angeordnete Vorrichtung zum Gefriertrocknen eingebracht wird, in der die Gefriertrocknung stattfindet, und dass der Behälter (11) nach der Gefriertrocknung aus der Vorrichtung entnommen und erneut in einen Reinraum eingeschleust wird.

7. Verfahren nach Anspruch 6, **dadurch gekennzeichnet, dass** die Vorrichtung zum Gefriertrocknen selbst nicht steril und/oder aseptisch ist.

8. Verfahren nach einem der vorhergehenden Ansprüche, **dadurch gekennzeichnet, dass** das Magazin (9) nach dem Verschließen der ersten Kammer (5) und vor dem Befüllen der zweiten Kammer (5') des mindestens einen Doppelkammersystems (3) gewendet wird.

9. Verfahren nach einem der vorhergehenden Ansprüche, **dadurch gekennzeichnet, dass** die erste Kammer (5) des mindestens einen Doppelkammersystems (3) mit einem Originalitätsverschluss verschließbar ist.

10. Verfahren nach einem der vorhergehenden Ansprüche, **dadurch gekennzeichnet, dass** die zweite Kammer (5) des mindestens einen Doppelkammersystems (3) mit einem Stopfen (23) verschließbar ist.

11. Verfahren nach Anspruch 10, **dadurch gekennzeichnet, dass** die zweite Kammer (5') des mindestens einen Doppelkammersystems mit einem Gewindestopfen (23) verschließbar st.

## Claims

1. Method for filling dual-chamber systems (3) in pre-sterilizable carrier systems (1), **characterized by** the following steps:
- providing at least one sterilized dual-chamber system (3) with respectively one separating element (7) separating the two chambers (5, 5') from one another in a magazine (9), which accommodates the at least one dual-chamber system (3), wherein the magazine (9) is arranged in a container (11) sealed with a closure element (13);
- introducing the container (11) into a clean room;
- opening the container (11) and filling a first chamber (5) of the at least one dual-chamber system (3);
- closing the first chamber (5) of the at least one dual-chamber system (3) with a gas-permeable closure element (19);
- freeze drying the solution (L1) contained in the first chamber (5) of the at least one dual-chamber system (3);
- closing the first chamber (5) of the at least one dual-chamber system (3) with a closure element (19);
- filling a second chamber (5') of the at least one dual-chamber system (3);
- closing the second chamber (5') of the at least one dual-chamber system (3);
- discharging from the clean room.

2. Method for filling dual-chamber systems (3) in pre-sterilizable carrier systems (1), **characterized by** the following steps:
- providing at least one sterilized dual-chamber system (3) with respectively one separating element (7) separating the two chambers (5, 5') from one another in a magazine (9), which accommodates the at least one dual-chamber system (3), wherein the magazine (9) is arranged in a container (11) sealed with a closure element (13);
- introducing the container (11) into a clean room;
- opening the container (11) and filling a first chamber (5) of the at least one dual-chamber system (3);
- closing the first chamber (5) of the at least one dual-chamber system (3) with a gas-permeable closure element (19);
- closing the container (11) with a gas-permeable closure element (13);
- freeze drying the solution (L1) contained in the first chamber (5) of the at least one dual-chamber system;
- closing the first chamber (5) of the at least one dual-chamber system (3) with a closure element (19);
- opening the container (11) and filling a second chamber (5') of the at least one dual-chamber system (3);
- closing the second chamber (5') of the at least one dual-chamber system (3);
- discharging from the clean room.

3. Method according to one of claims 1 or 2, **characterized in that** the magazine (9) comprises plastic, preferably is composed of plastic.

4. Method according to one of the preceding claims, **characterized in that** the container (11) comprises plastic, preferably is composed of plastic.

5. Method according to claim 4, **characterized in that** the plastic is elastically deformable.

6. Method according to one of claims 2 through 5, **characterized in that** after the filling of the first chamber (5) of the at least one dual chamber system (3) and the closure of the first chamber and of the container with a gas-permeable closure element (13), the container (11) is discharged from the clean room and is placed into a device for freeze drying arranged outside the clean room, in which device the freeze drying takes place, and that after the freeze drying the container (11) is removed from the device and is fed into a clean room again.

7. Method according to claim 6, **characterized in that** the device for freeze drying itself is not sterile and/or aseptic.

8. Method according to one of the preceding claims, **characterized in that** after the closure of the first chamber (5) and before the filling of the second chamber (5') of the at least one dual-chamber system (3), the magazine (9) is turned over.

9. Method according to one of the preceding claims, **characterized in that** the first chamber (5) of the at least one dual-chamber system (3) can be closed with a tamper-proof closure.

10. Method according to one of the preceding claims, **characterized in that** the second chamber (5') of the at least one dual-chamber system (3) can be closed with a plug (23).

11. Method according to claim 10, **characterized in that** the second chamber (5') of the at least one dual-chamber system can be closed with a screw plug (23).

## Revendications

1. Procédé de remplissage de systèmes à double chambre (3) dans des systèmes de support préstérilisables (1), **caractérisé par** les étapes suivantes :
- fourniture d'au moins un système à double chambre stérilisé (3) avec à chaque fois un élément de séparation (7) séparant les deux chambres (5, 5') l'une de l'autre dans un magasin (9) qui reçoit l'au moins un système à double chambre (3), dans lequel le magasin (9) est disposé dans un récipient (11) scellé par un élément de fermeture (13) ;
- introduction du récipient (11) dans une salle blanche ;
- ouverture du récipient (11) et remplissage d'une première chambre (5) de l'au moins un système à double chambre (3) ;
- fermeture de la première chambre (5) de l'au moins un système à double chambre (3) avec un élément de fermeture perméable au gaz (19) ;
- lyophilisation de la solution (L1) contenue dans la première chambre (5) de l'au moins un système à double chambre (3) ;
- fermeture de la première chambre (5) de l'au moins un système à double chambre (3) avec un élément de fermeture (19) ;
- remplissage d'une seconde chambre (5') de l'au moins un système à double chambre (3) ;
- fermeture de la seconde chambre (5') de l'au moins un système à double chambre (3) ;
- évacuation de la salle blanche.

2. Procédé de remplissage de systèmes à double chambre (3) dans des systèmes de support préstérilisables (1), **caractérisé par** les étapes suivantes :
- fourniture d'au moins un système à double chambre stérilisé (3) avec à chaque fois un élément de séparation (7) séparant les deux chambres (5, 5') l'une de l'autre dans un magasin (9) qui reçoit l'au moins un système à double chambre (3), dans lequel le magasin (9) est disposé dans un récipient (11) scellé par un élément de fermeture (13) ;
- introduction du récipient (11) dans une salle blanche ;
- ouverture du récipient (11) et remplissage d'une première chambre (5) de l'au moins un système à double chambre (3) ;
- fermeture de la première chambre (5) de l'au moins un système à double chambre (3) avec un élément de fermeture perméable au gaz (19) ;
- fermeture du récipient (11) avec un élément de fermeture perméable au gaz (13) ;
- lyophilisation de la solution (L1) contenue dans la première chambre (5) de l'au moins un système à double chambre ;
- fermeture de la première chambre (5) de l'au moins un système à double chambre (3) avec un élément de fermeture (19) ;
- ouverture du récipient (11) et remplissage d'une seconde chambre (5') de l'au moins un système à double chambre (3) ;
- fermeture de la seconde chambre (5') de l'au moins un système à double chambre (3) ;
- évacuation de la salle blanche.

3. Procédé selon l'une quelconque des revendications 1 ou 2, **caractérisé en ce que** le magasin (9) comprend du plastique, de préférence se compose de plastique.

4. Procédé selon l'une quelconque des revendications précédentes, **caractérisé en ce que** le récipient (11) comprend du plastique, de préférence se compose de plastique.

5. Procédé selon la revendication 4, **caractérisé en ce que** le plastique est déformable élastiquement.

6. Procédé selon l'une quelconque des revendications 2 à 5, **caractérisé en ce que** le récipient (11) est évacué de la salle blanche après le remplissage de la première chambre (5) de l'au moins un système à double chambre (3) et la fermeture de la première chambre et du récipient avec un élément de fermeture perméable au gaz (13) et introduit dans un dispositif disposé en dehors de la salle blanche pour la lyophilisation dans lequel la lyophilisation a lieu, et que le récipient (11) est prélevé du dispositif après la lyophilisation et de nouveau introduit dans une salle blanche.

7. Procédé selon la revendication 6, **caractérisé en ce que** le dispositif de lyophilisation n'est lui-même pas stérile et/ou aseptique.

8. Procédé selon l'une quelconque des revendications précédentes, **caractérisé en ce que** le magasin (9) est tourné après la fermeture de la première chambre (5) et avant le remplissage de la seconde chambre (5') de l'au moins un système à double chambre (3).

9. Procédé selon l'une quelconque des revendications précédentes, **caractérisé en ce que** la première chambre (5) de l'au moins un système à double chambre (3) peut être fermée par une fermeture d'originalité.

10. Procédé selon l'une quelconque des revendications précédentes, **caractérisé en ce que** la seconde chambre (5') de l'au moins un système à double chambre (3) peut être fermée par un bouchon (23).

11. Procédé selon la revendication 10, **caractérisé en ce que** la seconde chambre (5') de l'au moins un système à double chambre peut être fermée par un bouchon fileté (23).
